# EUROPEAN PATENT APPLICATION

(11) **EP 1 096 017 A2**
(43) Date of publication of application: **02.05.2001**
(21) Application number: 00123319.6
(22) Date of filing: 26.10.2000
(51) Int. Cl.: C12N 15/67

(54) **Method for increasing the production of physiologically active substances by cultured cells**

(30) Priority: 26.10.1999 JP 30327999
(71) Applicant: Welfide Corporation, Osaka-shi, Osaka 541-0046 (JP)
(72) Inventor: Tamashima, Hiroshi, Welfide Corporation Res. Labs., Hirakata-shi, Osaka 573-1153 (JP)
(74) Representative: VOSSIUS & PARTNER

(57) **Abstract**

The production of a physiologically active substance by a normal, transfected or transformed cultured cell can be increased by culturing the cell in a culture medium containing cobalt and molybdenum. The culture medium is one ordinarily selected for growing those types of cells which produce a desired, physiologically active substance, e.g., a recombinant growth factor of a monoclonal antibody. When cobalt and molybdenum are included in any of these growth media, a substantial improvement in the production of a physiologically active substance by the cells can be observed. Compositions and methods utilizing cobalt and molybdenum as additives for culture media are described.

## Description

The present invention relates to a method for culturing an animal cell capable of producing a physiologically active substance. More particularly, it relates to a method for improving productivity of a physiologically active substance in culturing an animal cell capable of producing the physiologically active substance.

With the recent advances in recombinant DNA technology, methods for producing physiologically active substances, such as recombinant proteins and the like, as expressed cellular products have been studied and developed. For the production of these cellular products, culturing of animal cells and cell lines is very important. Culture methods for increasing or enhancing the production of cellular products are important for scaling up production to meet industrial level needs.

Various culture media are available for growing (proliferating) and maintaining animal cells. Examples include basal culture media, such as Dulbecco's modified Eagle's medium ("DMEM"; *In Vitro*, *6*: 89 (1970)), F 12 medium (Proc. *Natl. Acad. Sci. USA*, *53*: 288 (1965)), RPMI 1640 medium (*J. Immunol. Methods*, *39*: 285 (1980); *JAMA*, *199*: 519 (1957)) or the like, a serum culture medium in which serum of bovine calf, horse, human or the like has been added to the basal culture medium, and a serum-free culture medium in which a substance other than serum has been added to the basal culture medium.

Examples of serum-free culture media include a culture medium containing insulin, peptone, transferrin and human serum albumin (JP-A-4-234982; the term "JP-A" as used herein means an "unexamined published Japanese patent application", U.S. Patent 5,389,538, or EP-A-541952), a culture medium containing insulin, peptone and a non-proteinous iron source (WO 98/00521), a culture medium containing recombinant insulin, plant peptone, inorganic iron and the like (U.S. Patent 5,316,938, U.S. Patent 5,633,162, or EP-A-481791), a culture medium containing recombinant human serum albumin (WO 98/06822), and the like.

On the other hand, methods in which various additives, such as succinic acid and the like (U.S. Patent 4,328,314 or GB-A-2037295), neutral amino acids and organic acids (JP-A-60-119189), butyric acid (JP-A-1-257492, U.S. Patent 5,681,718 or EP-A-239292), thioglycolic acid, cysteine and the like (ZA-A-8807596, IL-A-88001 or DE-A-3734632), are included as supplements in culture media, are recognized for improving the production of physiologically active substances by cultured animal cells.

An object of the present invention is to provide a method for improving the production of a physiologically active substance by a cultured animal cell or cell line which is capable of producing the physiologically active substance.

More specifically, the present invention relates to a method for improving the production of a physiologically active substance, comprising
adding cobalt and molybdenum to a culture medium for the production of a physiologically active substance obtainable by culturing an animal cell or cell line which is capable of producing the physiologically active substance in the culture medium.

Also, the present invention relates to a method for producing a physiologically active substance, comprising
culturing an animal cell or cell line which is capable of producing a physiologically active substance in a culture medium containing cobalt and molybdenum to produce the physiologically active substance; and
isolating the physiologically active substance from the culture medium.

This application is based on Japanese applications No. Hei 11-303279 filed on October 26, 1999, the entire content of which is incorporated hereinto by reference.

The present inventors have identified a method for improving the production of a physiologically active substance by adding cobalt and molybdenum to a culture medium for use in the production of the physiologically active substance by a cultured animal cell or cell line.

### A. Cobalt and molybdenum

Cobalt can be added to the culture medium in the form of cobalt chloride, cobalt acetate, cobalt sulfate, alum, or the like in the present invention. The amount of cobalt can range approximately from 0.1 to 50 µg/L depending on the particular culture medium.

Also, molybdenum can be added to the culture medium in the form of ammonium molybdate or the like in the present invention. The amount of molybdenum can range approximately from 0.1 to 100 µg/L depending on the particular culture medium.

### B. Culture medium

The culture medium of the present invention is not particularly limited, so long as it can sustain the production of animal cells. Culture media may include basal culture medium, serum culture medium and serum-free culture medium. Serum-free culture medium is preferred since it contains substantially no serum, making separation and purification of the physiologically active substance considerably easier. Specifically, the culture medium is composed of a basal culture medium and at least one of the additives described below.

### a. Basal culture medium

A basal culture medium generally used can also be used in the present invention. Examples include media containing a carbon source that can be assimilated by animal cells, a nitrogen source that can be digested thereby, inorganic salts and the like. Specific examples of a basal culture medium include MEM-α medium, DMEM medium, F 12 medium, RPMI 1640 medium, and the like. Among these, RPMI 1640 medium is particularly preferred.

### b. Additives

Examples of additives which can be used to supplement a basal culture medium include insulin, peptone, butyric acid or a salt thereof, iron, copper, zinc, manganese, and the like.

Sources of insulin include human, bovine, and the like. A recombinant or synthetic insulin as described in U.S. Patent 5,316,938, U.S. Patent 5,633,162 or EP-A-481791, is preferred. The amount of insulin can range approximately from 0.1 to 10 mg/L, and is more preferably approximately from 0.1 to 2 mg/L, depending on the culture medium.

The peptone may be derived from animal sources (e.g., Bacto-peptone, bovine, yolk, and the like) and plant sources (e.g., soybean, wheat, barley, and the like). A plant-derived peptone is preferred. The amount of peptone additive can range approximately from 0.1 to 50 g/L, and is more preferably approximately from 1 to 10 g/L, depending on the culture medium.

Other additives include butyric acid or salts thereof. Examples of a butyric acid salt include alkali metal salts (e.g., sodium salt, potassium salt, and the like), alkaline earth metal salts (e.g., calcium salt and the like), and the like. The amount of butyric acid additive can range approximately from 0.1 to 5 mM depending on the culture medium.

Iron may be either proteinous or non-proteinous, but is preferably non-proteinous. Specific examples include inorganic salts (e.g., sulfate, hydrochloride, nitrate, and the like), organic salts (e.g., acetate, citrate, and the like), and complex salts (e.g., EDTA complex salt, nitrosotriacetic acid complex salt, and the like). The amount of iron additive can range approximately from 0.01 to 100 mg/L, and is more preferably approximately from 0.1 to 50 mg/L, depending on the culture medium.

Copper can be added in the form of copper sulfate, copper chloride, copper acetate, copper nitrate, or the like. The amount of copper additive can range approximately from 0.1 to 100 µg/L depending on the culture medium.

Manganese can be added in the form of manganese sulfate, manganese chloride, manganese acetate, manganese nitrate, or the like. The amount of manganese additive can range approximately from 0.01 to 100 µg/L depending on the culture medium.

Zinc can be added in the form of zinc sulfate, zinc chloride, zinc acetate, zinc nitrate, or the like. The amount of zinc additive can range approximately from 0.01 to 100 µg/L depending on the culture medium.

Also, other conventional additives can be used as needed. Examples include hypoxanthine, thymidine, selenium, α-tocopherol (vitamin E) and the like. The amount of hypoxanthine can range approximately from 0.1 to 100 mg/L, and is more preferably approximately from 1 to 20 mg/L, depending on the culture medium. The amount of thymidine can range approximately from 0.01 to 100 mg/L, and is more preferably approximately from 1 to 10 mg/L, depending on the culture medium. The amount of selenium can range approximately from 0.01 to 100 µg/L, and is more preferably approximately from 1 to 10 µg/L, depending on the culture medium. The amount of α-tocopherol can range approximately from 0.001 to 10 mg/L, and is more preferably from 0.01 to 0.5 mg/L, depending on the culture medium.

Human serum albumin can also be added, and recombinant human serum albumin is preferable (WO 98/06822, EP-A-947581, U.S. Patent 5,962,649 or EP-A-699887). The amount of human serum albumin can range approximately from 0.1 to 5 g/L, and is more preferably approximately from 0.1 to 2 g/L, depending on the culture medium.

### C. Animal cell

The types of animal cells which can be cultured in the culture medium of the present invention include normal cells, transfected cells or transformed cells, or any cell which is capable of producing a physiologically active substance, *in vitro*.

A transfected cell is any cell having undergone a process of inserting foreign DNA into a culture of eukaryotic cells by exposing the cells to naked DNA.

A transformed cell can be either a cell having undergone a process of conversion to a state of unrestrained growth in culture, resembling or identical with the characteristics of cancerous growth, or a genetic change in a cell involving the incorporation into the cell of purified DNA from another cell.

Examples of normal cells which are capable of producing a physiologically active substance include an interferon ("IFN")-α producing leukocyte, a namalwa cell, a BALL-1 cell, an IFN-β producing fibroblast, an IFN-γ producing lymphocyte, a urokinase ("UK") or a precursor thereof ("proUK") producing human kidney cell, and the like.

Examples of transformed cells which are capable of producing a physiologically active substance include a monoclonal antibody-producing hybridoma and a tissue plasminogen activator-producing myeloma cell (e.g., Bowes cell or the like).

Examples of host cell lines which can be transfected by gene insertion to produce a physiologically active substance include Vero cells, Hela cells, CHO cells, WI 38 cells, BHK cells, COS-7 cells, MDCK cells, C 127 cells, human kidney cell lines, and the like. Specific examples include CHO-K₁ (Chinese hamster ovary cell, ATCC CCL61), BHK (hamster renal cell, ATCC CCL10), COS-7 (CV-1 origin, SV-40 cell, ATCC CRL1651), Vero cell (monkey (*Cerocopithecus*) kidney cell, ATCC CCL81), mouse myeloma cell (X63-Ag8-653, P3U1 or the like), human lymphoblast (IM-9, ATCC CCL159), parent cell for human hybridoma preparation, dihydrofolate reductase ("dhfr") deficient cell lines, hypoxanthine-guanine phosphoribosyltransferase (HGPRT) deficient cell lines, and ouabain resistant cell lines, and the like.

### D. Culturing method

The method for culturing an animal cell in the culture medium of the present invention can be carried out as follows. Although not limited to the type of culture vessel, a culture vessel such as a dish, a flask, a roller bottle, a spinner flask or a culture apparatus which uses microcarriers, microcapsules, or hollow fibers or the like, can be used for culturing an animal cell in the culture medium of the present invention.

In addition, the culture medium of the present invention can be used in subculturing methods such as a continuous culturing method in which a culture medium containing the cells or a culture medium collected during cell separation, is continuously or intermittently replaced with the equivalent amount of fresh culture medium. By constantly controlling the culturing conditions, cells are maintained in culture for prolonged periods of time.

The culture density of cells in the culture medium of the present invention can range approximately from 10⁴ to 10⁹ cells/ml. Also, animal cells can be cultured using the culture medium of the present invention under high density conditions such as 10⁸ cells/ml or more.

A culture temperature is preferably approximately from 10 to 37°C, more preferably approximately from 30 to 37°C. The culture period is preferably approximately from 1 to 10 days.

Production of physiologically active substances, such as proteins and the like, by animal cells can be carried out by well known methods such as those described in EP-A-154272, U.S. Patent 5,098,840, EP-A-253241, and the like.

Physiologically active substances include antithrombin III ("AT-III"), proUK, UK, TPA, hepatitis B virus antigen, various kinds of IFN, colony-stimulating factors, monoclonal antibodies, platelet membrane glycoprotein GPIb, and the like, and can be easily produced by cells cultured in the culture medium of the present invention. After completion of the culturing step, the physiologically active substance can be isolated from a culture supernatant, a filtrate or from cells by general separation and purification techniques.

According to the present invention, production of a physiologically active substance can be improved by adding cobalt and molybdenum to a culture medium for the production of physiologically active substances by culturing an animal cell capable of producing a physiologically active substance in the culture medium. Particularly, the preferred culture medium for culturing animal cells is a serum-free medium.

The present invention will be explained in more detail by the following examples, but the present invention is not limited thereto.

### Example 1

A basal medium for cell culturing (1 L) was prepared by mixing 10.4 g of RPMI 1640 medium (*J. Immunol. Methods*, *39*: 285 (1980); *JAMA*, *199*: 519 (1957)), 2 g of glucose, 2.5 g of sodium bicarbonate, 5 g of a soybean origin peptone (trade name "Soy Flour", manufactured by MDV), 1 mg of recombinant human insulin, 10 mg of ferrous sulfate and an appropriate amount of water to volume.

Thereafter, the basal medium was supplemented with 7 µg of cobalt chloride hexahydrate, 17 µg of copper sulfate pentahydrate, 159 µg of ferrous sulfate heptahydrate, 8 µg of ammonium molybdate tetrahydrate, 5 µg of manganese tetrahydrate and 10 µg of zinc sulfate heptahydrate. The media preparation is referred to as TM addition medium.

### Example 2

A GPIb-producing CHO cell was cultured in TM addition medium prepared in Example 1. This cell was obtained by transfecting CHO cells in the usual way with an expression plasmid containing a DNA insert encoding an α chain fragment (His(1) to Ala(302), molecular weight 45,000 daltons) of a platelet membrane surface glycoprotein GPIb. The expression plasmid ("pSV2-UK", EP-A-154272) contains a replication origin and an SV40 promoter in the upstream side and a ribosome binding region, an RNA splicing region, a poly(A) addition region and a transcription termination sequence in the downstream side. Furthermore, a methotrexate-containing selection medium was used.

The GPIb-producing CHO cell was inoculated into 10 ml of the basal culture medium (control) or TM addition medium (test) of Example 1 at a cell density of 12×10⁴ to 15×10⁴ cells/ml, and cultured at 37°C for 3 days.

The level of GPIb production was compared with between the test and control media. Transfected CHO cells cultured in TM addition medium of Example 1 produced 3.7 µg/ml of GPIb protein whereas cells grown under the control condition produced 2.2 µg/ml of the protein.

### Example 3

A culture medium was prepared by adding 33 µg of cobalt chloride hexahydrate, 75 µg of copper sulfate pentahydrate, 714 µg of ferrous sulfate heptahydrate, 38 µg of ammonium molybdate tetrahydrate, 24 µg of manganese tetrahydrate and 45 µg of zinc sulfate heptahydrate to 1 L of the basal culture medium in Example 1.

### Example 4

A hybridoma cell capable of producing a monoclonal antibody against blood coagulation factor IX was cultured in the medium of Example 3. This cell ("3A6") was prepared in accordance with the method disclosed in JP-A-1-258700.

### Example 5

A culture medium was prepared by mixing 1 L of a commercially available serum-free medium, SFMII medium (manufactured by GIBCO), with 60 µg of ZnSO₄·7H₂O, 32 µg of CuSO₄·5H₂O, 100 µg of MnSO₄·5H₂O, 44 µg of CoCl₂·6H₂O, and 50 µg of (NH₄)₆Mo₇O₂₄·4H₂O.

### Example 6

An AT-III-producing CHO cell was cultured in the medium of Example 5. This cell was obtained by selection of a dhfr deficient CHO-K₁ cell line into which a human AT-III gene and a dhfr gene had been inserted, using a low serum medium containing 0.5% (w/v) fetal bovine serum ("FBS") in accordance with the method disclosed in JP-A-4-349880 or *Biosci. Biotech. Biochem*., *56*: 600-604 (1992).

The AT-III-producing CHO cell was inoculated into 50 ml of the control SFMII medium or TM-SFMII medium of Example 5 at a cell density of 1×10⁵ cells/ml, and cultured at 37°C for 3 days in the presence of 5% CO₂.

Transfected CHO-K₁ cells cultured in TM addition medium of Example 5 produced 291 U/ml of the recombinant AT-III protein, whereas cells growth under the control condition produced 231 U/ml of the protein.

### Example 7

A culture medium was prepared by adding 10.1 g of α-MEM, 2.2 g of NaHCO₃, 100,000 units of penicillin, 100 mg of streptomycin, 2.5 ml of 2 mM methotrexate solution, 1 mg of insulin (manufactured by GIBCO), 5 g of soybean peptone ("SE50M", manufactured by Deltown), 1.16 mg of FeSO₄·7H₂O, TM components of 7.26 µg of CoCl₂·6H₂O, 16.5 µg of CuSO₄·5H₂O, 5.28 µg of MnSO₄·5H₂O, 8.25 µg of (NH₄)₆Mo₇O₂₄·4H₂O, and 9.9 µg of ZnSO₄·7H₂O and water to a final volume of 1 liter.

### Example 8

The AT-III-producing CHO cell disclosed in Example 6 was inoculated into 2 ml of the culture medium of Example 7 at a cell density of 2.5×10⁴ cells/ml, and cultured at 37°C for 2 to 7 days to produce a recombinant AT-III in the culture medium.

While the invention has been described in detail and with reference to specific embodiments thereof, it will be apparent to one of skill in the art that various changes and modifications can be made therein without departing from the spirit and scope thereof.

## Claims

1. A method for improving the production of a physiologically active substance, comprising
adding cobalt and molybdenum to a culture medium for the production of a physiologically active substance obtainable by culturing an animal cell or cell line which is capable of producing the physiologically active substance in the culture medium.

2. The method according to claim 1, wherein the culture medium comprises copper, iron, manganese or zinc.

3. The method according to claim 1 or 2, wherein the culture medium is serum-free.

4. The method according to any one of claims 1 to 3, wherein cobalt is added to the culture medium in an amount of 0.1 to 50 µg/L; and molybdenum is added to the culture medium in an amount of 0.1 to 100 µg/L.

5. The method according to any one of claims 1 to 4, wherein the physiologically active substance is selected from the group consisting of antithrombin III, a urokinase or a precursor thereof, a tissue plasminogen activator, a hepatitis B virus antigen, an interferon, a colony-stimulating factor, a monoclonal antibody, and platelet membrane glycoprotein GPIb.

6. A method for producing a physiologically active substance, comprising
culturing an animal cell or cell line which is capable of producing a physiologically active substance in a culture medium containing cobalt and molybdenum to produce the physiologically active substance; and
isolating the physiologically active substance from the culture medium.

7. The method according to claim 6, wherein the culture medium comprises copper, iron, manganese or zinc.

8. The method according to claim 6 or 7, wherein the culture medium is serum-free.

9. The method according to any one of claims 6 to 8, wherein cobalt is added to the culture medium in an amount of 0.1 to 50 µg/L; and molybdenum is added to the culture medium in an amount of 0.1 to 100 µg/L.

10. The method according to any one of claims 6 to 9, wherein the physiologically active substance is selected from the group consisting of antithrombin III, a urokinase or a precursor thereof, a tissue plasminogen activator, a hepatitis B virus antigen, an interferon, a colony-stimulating factor, a monoclonal antibody, and platelet membrane glycoprotein GPIb.
